# EUROPÄISCHE PATENTANMELDUNG

(11) **EP 0 637 581 A1**
(43) Veröffentlichungstag der Anmeldung: **08.02.1995**
(21) Anmeldenummer: 94114552.6
(22) Anmeldetag: 21.01.1992
(51) Int. Cl.: C07C 51/54, C07D 319/06, C07C 55/08

(54) **Verwendung von Malonsäureanhydrid zur Herstellung von 2,2-disubstituierten 4,6-dioxo-1,3-dioxanen**

(30) Priorität: 21.01.1991 CH 156/91; 21.01.1991 CH 157/91
(62) Teilanmeldung aus: 92100948.6
(71) Anmelder: LONZA AG, CH-3945 Gampel/Wallis (CH)
(72) Erfinder: O'Murchu, Colm, Dr., Visp (Kanton Wallis) (CH)
(74) Vertreter: Weinhold, Peter, Dr.

(57) **Zusammenfassung**

Die Erfindung betrifft die Verwendung von Malonsäureanhydrid zur Herstellung von 2,2-disubstituierten 4,6-Dioxo-1,3-dioxanen bzw. ein Verfahren zur Herstellung der Dioxane. Die Herstellung der Dioxane erfolgt durch Umsetzung von Malonsäureanhydrid mit einem Keton der Formel R₃C(O)R₄, in der R₃ und R₄ Alkyl- oder Arylgruppen bedeuten oder zusammen ein gegebenenfalls substituiertes cyclisches System bilden, in Gegenwart einer Lewis-Säure oder einer protischen Säure.

## Beschreibung

Die Erfindung betrifft die Verwendung von Malonsäureanhydrid zur Herstellung von 2,2-di-substituierten 4,6-Dioxo-1,3-dioxanen bzw. ein Verfahren zur Herstellung der Dioxane.

Die Verwendung von Malonsäureanhydrid bei der Herstellung 2,2-disubstituierter 4,6-Dioxo-1,3-dioxane ermöglicht die Umsetzung in wirtschaftlicher Weise und im technischen Maßstab.

Genauer gesagt, betrifft die vorliegende Erfindung die Verwendung von Malonsäureanhydrid der Formel 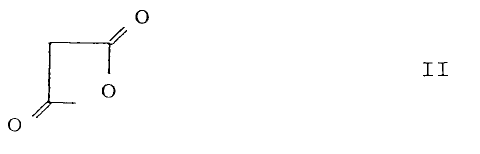
zur Herstellung von 2,2-disubstituierten 4,6-Dioxo-1,3-dioxanen der allgemeinen Formel 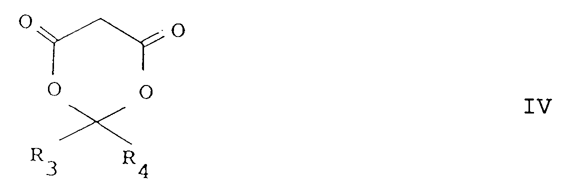
worin R₃ und R₄ gleich oder verschieden sind und eine Alkylgruppe oder eine Arylgruppe bedeuten oder worin R₃ und R₄ zusammen ein gegebenenfalls substituiertes cyclisches System bilden, durch Umsetzung des Malonsäureanhydrids bei einer Temperatur zwischen -30°C und -100°C mit einem Keton der allgemeinen Formel 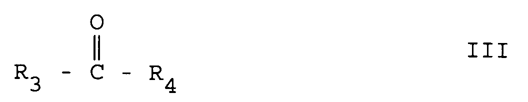
worin R₃ und R₄ die genannte Bedeutung haben, in Gegenwart einer Lewis-Säure oder einer protischen Säure.

Weiterhin betrifft die Erfindung ein Verfahren zur Herstellung von 2,2-disubstitutierten 4,6-Dioxo-1,3-dioxanen der allgemeinen Formel 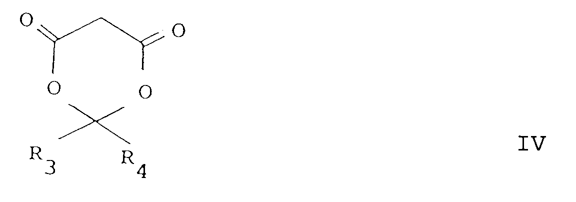
worin R₃ und R₄ gleich oder verschieden sind und eine Alkylgruppe oder eine Arylgruppe bedeuten oder worin R₃ und R₄ zusammen ein gegebenenfalls substituiertes cyclisches System bilden, durch Umsetzung von Malonsäureanhydrid der Formel 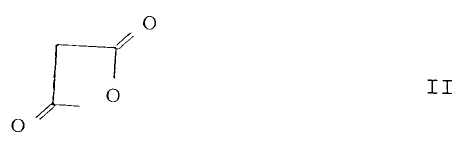
bei einer Temperatur zwischen -30°C und -100°C mit einem Keton der allgemeinen Formel 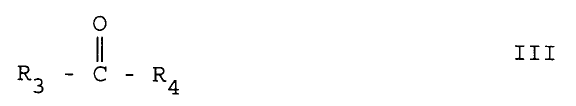
worin R₃ und R₄ die genannte Bedeutung haben, in Gegenwart einer Lewis-Säure oder einer protischen Säure.

Das erfindungsgemäß eingesetzte Malonsäureanhydrid kann beispielsweise durch Ozonisierungen von Diketen hergestellt werden.

So beansprucht Perrin in der US-PS 4 251 447 und der US-PS 4 360 691 die Herstellung von Malonsäureanhydrid bzw. von Malonsäureanhydridderivaten durch Umsetzung von Diketen bei 0°C bis -100°C mit Ozon. Ein aus Sicherheitsgründen schwerwiegender Nachteil dieses Verfahrens ist aber, dass als Nebenprodukt in organischen Lösungsmitteln unlösliche Formaldehydperoxid-Polymere entstehen (US-PS 4 360 691, Spalte 5, Zeile 3-5), die z.B. gemäss Lapalme et al., Can.J.Chem. 57, S.3272 (1979) hochexplosiv sind.

Vorteilhaft erfolgt die Herstellung des Malonsäureanhydrids nach dem in EP-A-92 100 948 beschriebenen Verfahren. Hierbei wird großtechnisch verfügbares Diketen zunächst bei einer Temperatur zwischen -30°C und -100°C in Gegenwart einer Carbonylverbindung der allgemeinen Formel 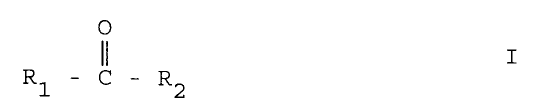
worin R₁ und R₂ gleich oder verschieden sind und Wasserstoff, eine Alkylgruppe, eine Alkoxygruppe oder eine Arylgruppe bedeuten, zum Malonsäureanhydrid der Formel 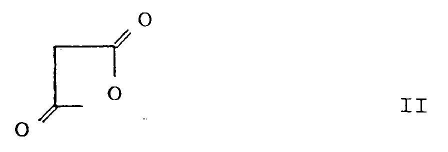
ozonisiert.

Da sich das Malonsäureanhydrid bei Temperaturen von -30°C und höher in Keton und CO₂ zersetzt, wird es in der Regel direkt mit den Ketonen, insbesondere mit Aceton, zu den entsprechenden 2,2-disubstituierten 4,6-Dioxo-1,3-dioxanen umgesetzt.

Das Malonsäureanhydrid muß jedoch nicht unbedingt direkt weiter umgesetzt werden, sondern kann auch bei Temperaturen unter -30°C, insbesondere bei Temperaturen unter -40°C, konserviert werden.

Die Ozonisierung in Gegenwart einer der genannten Carbonylverbindungen bewirkt, dass anstelle der Bildung der Formaldehydperoxid-Polymere lösliche Ozonide gebildet werden, die gefahrlos abgetrennt werden können. Dies geschieht zweckmäßig nach erfolgter Weiterumsetzung bei der Aufarbeitung der Malonsäurederivate. Im Falle der Umsetzung mit Ketonen erfolgt die Aufarbeitung und Trennung vorteilhaft mittels Chromatographie der Produkte.

Bei den Resten R₁ und R₂ bedeutet Alkyl zweckmässig eine Niederalkylgruppe mit 1-4 C-Atomen, vorzugsweise eine Methyl-, Ethyl-, Propyl-, n-Butyl-, Isobutyl- oder tert-Butylgruppe. Alkoxy bedeutet zweckmässig eine Methoxy- oder Ethoxygruppe. Aryl bedeutet zweckmässig Phenyl oder p-Tolyl, vorzugsweise Phenyl.

Zweckmässig werden als Carbonylverbindungen Aldehyde der allgemeinen Formel 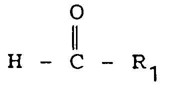
worin R₁ die genannte Bedeutung hat, eingesetzt.

Bevorzugte Aldehyde sind Formaldehyd, Acetaldehyd, Propionaldehyd, Isobutyraldehyd, Pivalaldehyd, Benzaldehyd oder p-Tolylaldehyd. Besonders bevorzugt wird Acetaldehyd eingesetzt.

Zweckmässig werden die Carbonylverbindungen stöchiometrisch oder im leichten Überschuss, bezogen auf Diketen, eingesetzt.

Die Reaktionstemperatur bewegt sich zwischen -30°C und -100°C. Da, wie eingangs erwähnt, sich das Malonsäureanhydrid bei -30°C zu zersetzen beginnt, wird vorzugsweise bei einer Temperatur unter -30°C, vorzugsweise zwischen -40°C und -80°C, gearbeitet.

Von Vorteil erfolgt die Reaktion in Gegenwart von aprotischen, wasserfreien Lösungsmitteln, wie z.B.
in Alkanen,z.B. Pentan, Hexan;
in aromatischen Kohlenwasserstoffen, z.B. C₁-C₄-alkylsubstituierten Benzolen,wie z.B. Toluol, Ethylbenzol, Dimethylbenzol, Ethylmethylbenzol, Propylbenzol, Isopropylbenzol, Butylbenzol, Isobutylbenzol, Diethylbenzol, Mesitylen, Cymol; halogenierten Benzolen,wie z.B. Fluorbenzol, Chlorbenzol;
in Ethern, z.B. Dimethylether, Diethylether, Dipropylether, Dibutylether, tertiär-Butylmethylether, Tetrahydrofuran, Dioxan; in Estern,z.B. C₁-C₈-Alkylformiaten,wie Methylformiat, Ethylformiat, Propylformiat, Isobutylformiat, Hexylformiat, Octylformiat; C₁-C₈-Alkylacetaten,wie Methylacetat, Ethylacetat, Propylacetat, Butylacetat, 2-Ethylhexylacetat, Pentylacetat, Hexylacetat, Heptylacetat, Octylacetat;
in Nitrilen,z.B. Acetonitril, Propionitril, Butyronitril oder in halogenierten Kohlenwasserstoffen,z.B. Methylenchlorid, Chloroform.

In der Regel wird so vorgegangen, dass man Ozon, welches als Gasgemisch mit Hauptanteil Sauerstoff generiert wird, in eine Lösung von Diketen und entsprechender Carbonylverbindung in einem der genannten Lösungsmittel so lange einleitet, bis Blaufärbung der Lösung das Ende der Ozonisierung anzeigt oder bis die stöchiometrische Menge Ozon absorbiert ist.

Zur erfindungsgemässen Herstellung der 2,2-disubstituierten 4,6-Dioxo-1,3-dioxane wird also Malonsäureanhydrid der Formel 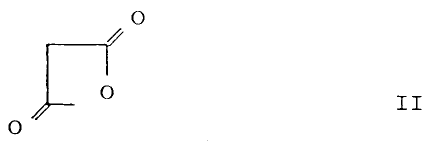
mit einem Keton der allgemeinen Formel 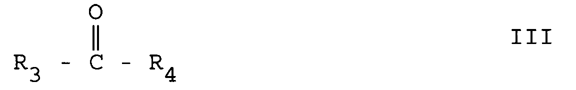
worin R₃ und R₄ gleich oder verschieden sind und eine Alkylgruppe oder eine Arylgruppe bedeuten oder R₃ und R₄ zusammen ein gegebenenfalls substituiertes cyclisches System bilden, in Gegenwart einer Lewis-Säure oder einer protischen Säure zum entsprechenden 2,2-disubstituierten 4,6-Dioxo-1,3-dioxanderivat der allgemeinen Formel 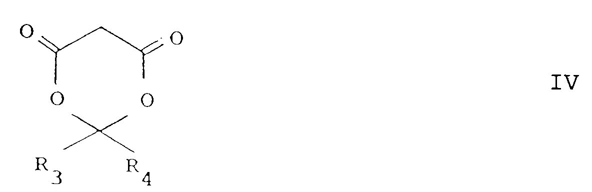
worin R₃ und R₄ die genannte Bedeutung haben, umgesetzt.

Bei den Resten R₃ und R₄ bedeutet Alkyl zweckmässig eine Niederalkylgruppe mit 1 bis 6 C-Atomen, bevorzugt eine Methyl- oder Ethylgruppe. Aryl bedeutet zweckmässig eine Phenyl- oder eine substituierte Phenylgruppe, vorzugsweise eine Phenylgruppe. Geeignete Ketone sind entsprechend z.B. Aceton, Methylethylketon oder Methylisobutylketon. Als cyclische Ketone können beispielsweise Cyclohexanon, Cyclopentanon, Campher oder Carvon eingesetzt werden. Besonders geeignete Ketone sind Aceton, Methylethylketon oder Acetophenon.

Von Vorteil erfolgt die Reaktion in Gegenwart der gleichen aprotischen, wasserfreien Lösungsmittel, wie sie bereits oben im Zusammenhang mit der Ozonisierung von Diketen genannt worden sind.

Erfolgt die Herstellung des Malonsäureanhydrids gemäß dem in EP-A-92 100 948 beschriebenen Verfahren, wird die Umsetzung mit dem Keton zweckmäßig in Gegenwart des bei der Ozonisierung verwendeten aprotischen, wasserfreien Lösungsmittels durchgeführt, besonders bevorzugt ohne vorherige Isolierung des Malonsäureanhydrids.

Pro Mol Diketen werden zweckmässig 0,5 bis 10 Mol, vorzugsweise zwischen 1 Mol und 4 Mol des betreffenden Ketons eingesetzt.

Die Reaktionstemperatur bewegt sich erfindungsgemäss zwischen -30°C und -100°C, vorzugsweise unter der Zersetzungstemperatur des Malonsaureanhydrids von -30°C, zwischen unter -30°C und -50°C.

Als Lewis-Säuren kommen die in der Fachwelt bekannten Verbindungen wie z.B. Eisen-, Bor-, Aluminium-, Zinn-, Titan-, Zink-, Antimon- oder Siliciumhalogenide in Betracht. Vorzugsweise werden Zinntetrachlorid oder Bortrifluorid-etherat angewendet. Als protische Säuren kommen zweckmässig Schwefelsäure, Trifluoressigsäure, Phosphorsäure oder Salzsaure in Frage.

Die Lewis-Säuren oder protischen Säuren werden zweckmässig in einer Menge zwischen 0,001 Mol und 0,5 Mol, vorzugweise zwischen 0,005 Mol und 0,05 Mol pro Mol Diketen, eingesetzt.

Die genannte Umsetzung, ausgehend von Diketen, verläuft üblicherweise innerhalb von ca. 1 h bis 1,5 h.

Die resultierenden 2,2-disubstituierten 4,6-Dioxo-1,3-dioxane können auf fachmännisch übliche Weise isoliert werden.

Die folgenden Beispiele illustrieren die Herstellung von Meldrumsäure (2,2-Dimethyl-4,6-dioxo-1,3-dioxan) und anderen 2,2-disubstituierten 4,6-Dioxo-1,3-dioxanen sowie ein besonders vorteilhaftes Verfahren zur Herstellung des Ausgangsmaterials Malonsäureanhydrid.

### Verfahren zur Herstellung von Malonsäureanhydrid

### Beispiel 1:

Eine Lösung von 4,2 g (0,050 Mol) Diketen und 2,2 g (0,050 Mol) Acetaldehyd in 100 ml Chloroform wurde bei -60°C mit einem Strom 4%igen Ozons in Sauerstoff bis zur Blaufärbung der Lösung ozonisiert.
Das überschüssige Ozon wurde zuerst mit Sauerstoff, dann mit Stickstoff gespült. Charakterisierung des Reaktionsproduktes:
¹H-NMR: (Reaktionsgemisch bei -50°C, 300 MHz) δ in ppm:
- Malonsäureanhydrid: 4,24, s
- Propylenozonid:: 1,52, d, CH₃,
5,1, s, CH,
5,31, q, CH,
5,32, s, CH.

### Beispiel 2

### Herstellung von Meldrumsäure (2,2-Dimethyl-4,6-dioxo-1,3-dioxan)

Eine Lösung von 4,2 g (0,050 Mol) Diketen und 2,2 g (0,050 Mol) Acetaldehyd in 100 ml Chloroform wurde bei -60°C mit einem Strom 4%igen Ozons in Sauerstoff bis zur Blaufärbung der Lösung ozonisiert.
Das überschüssig Ozon wurde zuerst mit Sauerstoff, dann mit Stickstoff gespült.
Zur kalten (-60°C) klaren Lösung wurde eine Lösung aus 0,13 g Zinntetrachlorid in 5,8 g (0.100 Mol) Aceton zugegeben und bei -40°C während 15 min weitergerührt.

Die klare Lösung wurde bei Raumtemperatur unter vermindertem Druck auf ca. 20 ml eingeengt und durch eine Silicagelsäule mit Chloroform/Tetrahydrofuran (98:2) als Eluat chromatographiert. Die Fraktionen die das Produkt enthielten, wurden unter vermindertem Druck auf 100 ml eingeengt und mit Wasser bei pH 9 bis 12,5 extrahiert. Die wässrige Phase wurde wieder auf pH 2 angesäuert und mit Methylenchlorid extrahiert. Nach Eindampfen des Lösungsmittels erhielt man als Rückstand 5,0 g farblose Meldrumsäure.
Smp. 93-94°C.

### Beispiel 3

### Herstellung von 2-Methyl-2-ethyl-1,3-dioxan-4,6-dion

Eine Lösung von 4,2 g (0,050 Mol) Diketen und 2,2 g (0,050 Mol) Acetaldehyd in 100 ml Chloroform wurde bei -60°C mit einem Strom 4%-igen Ozons in Sauerstoff bis zur Blaufärbung der Lösung ozonisiert. Das überschüssige Ozon wurde zuerst mit Sauerstoff, dann mit Stickstoff gespült. Zur kalten (-60°C), klaren Lösung wurde eine Lösung aus 0,13 g Zinntetrachlorid in 7,21 g (0,100 Mol) Methyl-ethyl-keton zugegeben und bei -40°C während einer Stunde weitergerührt. Die klare Lösung wurde bei Raumtemperatur unter vermindertem Druck auf ca. 20 ml eingeengt und durch eine Silicagelsäule mit Chloroform/Tetrahydrofuran (98:2) als Eluat chromatographiert. Die Fraktionen, die das Produkt enthielten, wurden unter vermindertem Druck auf 100 ml eingeengt und mit Wasser bei pH 12,3 extrahiert. Die wässrige Phase wurde wieder auf pH 2,3 angesäuert und mit Chloroform extrahiert. Nach Eindampfen des Lösungsmittels erhielt man als öligen Rückstand 5,0 g farbloses 2-Methyl-2-ethyl-1,3-dioxan-4,6-dion.
- ¹H-NMR (CDCl₃, 300 MHz) δ in ppm:: 1,08, t, CH₃,
1,73, s, CH₃,
2,01, q, CH₂,
3,62, s, CH₂.

### Beispiel 4

### Herstellung von 2-Methyl-2-phenyl-1,3-dioxan-4,6-dion

Eine Lösung von 4,2 g (0.050 Mol) Diketen und 2,2 g (0,050 Mol) Acetaldehyd in 100 ml Chloroform wurde bei -60°C mit einem Strom 4%-igen Ozons in Sauerstoff bis zur Blaufärbung der Lösung ozonisiert. Das überschüssige Ozon wurde zuerst mit Sauerstoff, dann mit Stickstoff gespült. Zur kalten (-60°C) klaren Lösung wurde eine Lösung aus 0,13 g Zinntetrachlorid in 12,13 g (0,100 Mol) Acetophenon zugegeben und bei -40°C während einer Stunde weitergerührt. Die klare Lösung wurde bei Raumtemperatur und 60 mbar auf ca. 20 ml eingeengt und durch eine Silicagelsäule mit Chloroform/Tetrahydrofuran (98:2) als Eluat chromatographiert. Die produktentahltenden Fraktionen wurden unter vermindertem Druck auf 100 ml eingeengt und mit Wasser bei pH 11,7 extrahiert. Die wässrige Phase wurde wieder auf pH 2,3 angesäuert und mit Chloroform extrahiert.

Nach Eindampfen des Lösungsmittels erhielt man 7,15 g 2-Methyl-2-phenyl-1,3-dioxan-4,6-dion als farbloses kristallines Pulver.
- ¹H-NMR (CDCl₃, 300 MHz) δ in ppm:: 1,97, s, CH₃,
3,00, d, CH,
3,44, d, CH,
7,4-7,5, m, Ph.

## Patentansprüche

1. Verwendung von Malonsäureanhydrid der Formel 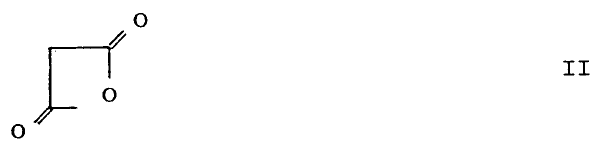 zur Herstellung von 2,2-disubstituierten 4,6-Dioxo-1,3-dioxanen der allgemeinen Formel 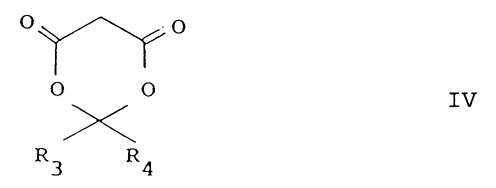 worin R₃ und R₄ gleich oder verschieden sind und eine Alkylgruppe oder eine Arylgruppe bedeuten oder worin R₃ und R₄ zusammen ein gegebenenfalls substituiertes cyclisches System bilden, durch Umsetzung des Malonsäureanhydrids bei einer Temperatur zwischen -30°C und -100°C mit einem Keton der allgemeinen Formel 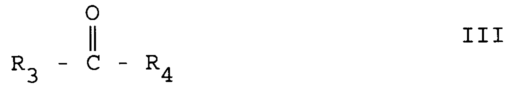 worin R₃ und R₄ die genannte Bedeutung haben, in Gegenwart einer Lewis-Säure oder einer protischen Säure.

2. Verfahren zur Herstellung von 2,2-disubstitutierten 4,6-Dioxo-1,3-dioxanen der allgemeinen Formel 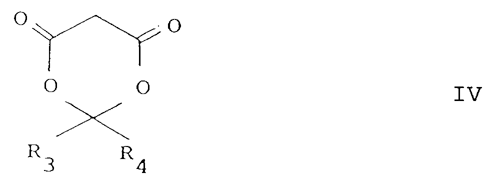 worin R₃ und R₄ gleich oder verschieden sind und eine Alkylgruppe oder eine Arylgruppe bedeuten oder worin R₃ und R₄ zusammen ein gegebenenfalls substituiertes cyclisches System bilden, durch Umsetzung von Malonsäureanhydrid der Formel 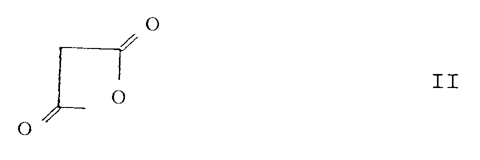 bei einer Temperatur zwischen -30°C und -100°C mit einem Keton der allgemeinen Formel 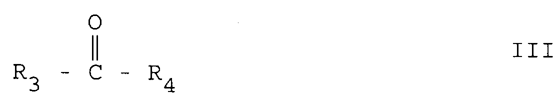 worin R₃ und R₄ die genannte Bedeutung haben, in Gegenwart einer Lewis-Säure oder einer protischen Säure.

3. Verfahren nach Patentanspruch 2, dadurch gekennzeichnet, daß das Malonsäureanhydrid durch Ozonisierung von Diketen hergestellt worden ist, vorzugsweise indem Diketen bei einer Temperatur zwischen -30°C und -100°C in Gegenwart einer Carbonylverbindung der allgemeinen Formel 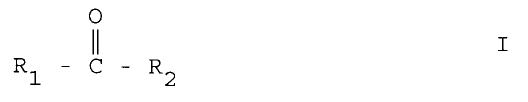 worin R₁ und R₂ gleich oder verschieden sind und Wasserstoff, eine Alkylgruppe, eine Alkoxygruppe oder eine Arylgruppe bedeuten, ozonisiert wird.

4. Verfahren nach Patentanspruch 3, dadurch gekennzeichnet, daß das Malonsäureanhydrid direkt ohne Isolierung mit dem entsprechenden Keton umgesetzt wird.

5. Verfahren nach mindestens einem der Patentansprüche 2 bis 4, dadurch gekennzeichnet, daß die Umsetzung mit dem entsprechenden Keton bei einer Temperatur zwischen unter -30°C und -50°C, zweckmäßig in Gegenwart des bei der Ozonisierung verwendeten aprotischen, wasserfreien Lösungsmittels, durchgeführt wird.

6. Verfahren nach mindestens einem der Patentansprüche 2 - 5, dadurch gekennzeichnet, daß die Lewis-Säure oder die protische Säure in einer Menge von 0,001 Mol bis 0,5 Mol pro Mol Diketen eingesetzt wird.

7. Verfahren nach mindestens einem der Patentansprüche 2 bis 6, dadurch gekennzeichnet, daß ein Keton der genannten allgmeinen Formel III, worin R₃ und R₄ gleich oder verschieden sind und eine C₁-C₆-Niederalkylgruppe oder eine Phenylgruppe bedeuten, eingesetzt wird.
